(19) Europäisches Patentamt
European Patent Office
Office européen des brevets

(11) **EP 1 415 682 B1**

(12) **EUROPÄISCHE PATENTSCHRIFT**

(45) Veröffentlichungstag und Bekanntmachung des Hinweises auf die Patenterteilung:
**07.01.2015 Patentblatt 2015/02**

(51) Int Cl.:
***A61N 1/37*** *(2006.01)*

(21) Anmeldenummer: **03090342.1**

(22) Anmeldetag: **13.10.2003**

(54) **Stimulationsvorrichtung mit Stimulationserfolgskontrolle**

Stimulation apparatus with capture verification

Dispositif de stimulation avec verification de capture

(84) Benannte Vertragsstaaten:
**AT BE BG CH CY CZ DE DK EE ES FI FR GB GR HU IE IT LI LU MC NL PT RO SE SI SK TR**

(30) Priorität: **30.10.2002 DE 10250996**

(43) Veröffentlichungstag der Anmeldung:
**06.05.2004 Patentblatt 2004/19**

(73) Patentinhaber: **BIOTRONIK SE & Co. KG**
**12359 Berlin (DE)**

(72) Erfinder: **Czygan, Gerlad, Dr. Ing.**
**91054 Buckenhof (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
**DE-A- 4 126 363    US-A- 5 431 693**
**US-A- 5 766 230    US-B1- 6 226 551**

**EP 1 415 682 B1**

**Beschreibung**

Die Erfindung betrifft eine Vorrichtung zur Elektrostimulation von Körpergewebe, insbesondere einen Herzschrittmacher. Die Elektrostimulationsvorrichtung weist eine Energiequelle zur Bereitstellung elektrischer Stimulationsenergie, ein Elektroden-Anschluss zum Anschließen einer Stimulationselektrode für die Abgabe von elektrischen Stimulationsimpulsen und einen Schalter auf, mit welchem die Energiequelle zur Abgabe eines Stimulationsimpulses mit dem Elektroden-Anschluss verbunden ist. Weiterhin weist die Elektrostimulationsvorrichtung eine Einrichtung zur Stimulationserfolgskontrolle auf. Darüber hinaus umfasst die Elektrostimulationsvorrichtung einen Kurzschlussschalter, mit dem Elektroden-Anschluss nach Abgabe des Stimulationsimpulses zumindest mittelbar mit einem Massepotential so zu verbinden ist, dass sich im Falle einer angeschlossenen und implantierten Elektrodenleitung eine Kapazität über das Körpergewebe und eine Masse-Elektrode derart entladen kann, dass ein Kurzschlussstrom durch das Körpergewebe fließt, wobei die Kapazität wenigstens eine Helmholz-Kapazität einschließt, welche sich auf der Oberfläche der Stimulationselektrode in Verbindung mit umgebender Körperflüssigkeit oder Körpergewebe einstellt. Auch umfasst die Elektrostimulationsvorrichtung eine Steuereinheit, die zumindest mit dem ersten Schalter und dem Kurzschlussschalter zum Umschalten dieser Schalter verbunden und ausgebildet ist, den Elektrodenleitungsanschluss nach Abgabe des Stimulationsimpulses von der Energiequelle zu trennen und zumindest indirekt mit dem Massepotential zu verbinden.

Elektrostimulationsvorrichtungen der vorgenannten Art sind insbesondere als implantierbare Herzschrittmacher bereits bekannt. Solche Herzschrittmacher sind im implantierten und funktionsfähigen Zustand üblicherweise über eine Elektrodenleitung der vorgenannten Art mit einer im Herzen angeordneten Elektrode verbunden und ausgebildet, über die Elektrode elektrische Stimulationsimpulse an das Herz abzugeben. Diese Stimulationsimpulse dienen der Erregung des Herzgewebes oder Myokards und werden je nach Herzschrittmacherart insbesondere dann abgegeben, wenn sich das Herz nicht zur rechten Zeit auf natürliche Weise kontrahiert. Eine Kontraktion wird dann durch einen elektrischen Impuls hervorgerufen, der an das Myokard abgegeben wird. Dieser elektrische Impuls hat - wenn er ausreichend bemessen ist - die Wirkung, dass das Herzmuskelgewebe örtlich depolarisiert wird und entsprechend kontrahiert. Die Depolarisation und Kontraktion des Herzmuskelgewebes soll sich über den gesamten stimulierten Herzmuskel ausdehnen und so zur gewünschten Kontraktion der entsprechenden Herzkammer führen.

Der entsprechende elektrische Stimulationsimpuls muss eine Stimulationsintensität besitzen, die über einer jeweiligen Reizschwelle des Herzmuskelgewebes liegt. Die Reizschwelle ist dabei ein Maß für die Mindeststimulationsintensität, die ausreicht, um eine Depolarisation des Myokards und damit einer Kontraktion einer jeweiligen Kammer des Herzens zu bewirken. Die Reizschwelle hängt von verschiedenen Faktoren ab und ist darüber hinaus im Laufe der Zeit unter Umständen auch veränderlich. Neben dem Erfordernis, einen Stimulationsimpuls ausreichender Stimulationsintensität abzugeben, besteht das Bedürfnis, die für einen Stimulationsimpuls aufzuwendende Energie so gering wie möglich zu halten. Diese Energie wird regelmäßig einer Batterie des Herzschrittmachers entnommen, die sich im Laufe der Zeit erschöpft. Wenn diese Batterie erschöpft ist, muss ein implantierter Herzschrittmacher in einer Operation durch einen neuen Herzschrittmacher ersetzt werden. Eine möglichst lange Betriebsdauer des Herzschrittmachers und damit eine möglichst lange Batterielebensdauer ist somit wünschenswert. Die Energie für einen Stimulationsimpuls soll außerdem auch deshalb möglicht gering, aber ausreichend sein, um nur das Myocard, aber nicht umliegendes Muskelgewebe zu stimulieren.

Es besteht somit einerseits das Erfordernis, dass die Stimulationsintensität eines Stimulationsimpulses ausreichen muss, eine Kontraktion des Herzmuskelgewebes auszulösen. Eine höhere Stimulationsintensität geht beim übrigen unveränderten Einflussgrößen mit einem größeren Energieverbrauch einher. Die Stimulationsintensität hängt zum einen von der Dauer eines Stimulationsimpulses und andererseits von der Stärke eines Stimulationsimpulses ab. Die Stärke eines Stimulationsimpulses wiederum hängt von der elektrischen Spannung ab, mit der ein Stimulationsimpuls an das Herzmuskelgewebe abgegeben wird. Daher führt eine höhere Stimulationsintensität regelmäßig auch zu einem größeren Energieverbrauch. Um eine sichere Stimulation des Herzmuskelgewebes zu erreichen, werden regelmäßig Stimulationsimpulse abgegeben, die in energetischer Hinsicht etwas mehr Energie kosten, als minimal erforderlich wäre.

Andererseits besteht das Bedürfnis, den Energieverbrauch pro Stimulationsimpuls so gering wie möglich zu halten, da diese Energie einer Batterie des Herzschrittmachers entnommen wird, die auf diese Weise erschöpft wird.

Es besteht somit das Bedürfnis, die Anforderungen nach einer möglichst geringen Stimulationsintensität und gleichzeitig einer regelmäßigen erfolgreichen Stimulation durch Optimieren der Stimulationsintensität zu erfüllen. Hierzu ist es aus dem Stand der Technik beispielsweise aus den US-Patenten 5,350,410, US 5,411,533, US 5,431,639 und US 5,674,254 bekannt, nach Abgabe eines Stimulationsimpulses den Stimulationserfolg (capture) zu erfassen (capture recognition) um möglichst bei mangelhaften Stimulationserfolg einen Back-up-Stimulationsimpuls auszulösen.

Es ist auch bekannt, einen Stimulationserfolg durch Erfassen der Impedanz des Herzmuskelgewebes zu detektieren. In diesem Zusammenhang sei auf die US-Patente 5,713,933 und US 5,735,883 und US 5,766,230 sowie auf die europäische Patentanmeldung 0 399 063 verwiesen. US 6,226,551 detektiert den Stimulationserfolg durch Erfassung der vom Herzen nach Abgabe des Stimulationsimpulses erzeugten elektrischen Signale.

Trotz der vielen bekannten Lösungsvorschläge zur Stimulationserfolgskontrolle und zur automatischen Anpassung der Stimulationsimpulsintensität besteht nach wie vor das Bedürfnis nach einer in dieser Hinsicht zuverlässigen Vorrichtung.

Der Erfindung liegt die Aufgabe zugrunde, eine geeignete Vorrichtung zumindest als Alternative zum bekannten Stand der Technik zu bieten.

Erfindungsgemäß wird diese Aufgabe durch eine elektrische Stimulationsvorrichtung gemäß Anspruch 1 gelöst. Die Erfindung zielt also darauf ab, eine während der Abgabe eines Stimulationsimpulses geladene Kapazität - sei es die Helmholzkapazität, die sich auf der Oberfläche des Stimulationselektrode ausbildet oder eine andere Kapazität - nach Abgabe des Stimulationsimpulses über das Herzmuskelgewebe kurz zu schließen und die entsprechende Kurzschlussspannung oder den Kurzschlussstrom oder eine hiervon abgeleitete Größe zur Bestimmung der Myokard-Impedanz zu erfassen.

Ein Vorteil dieser Stimulationserfolgskontrolle ist, dass das Ergebnis der Erfolgskontrolle in sehr kurzer Zeit erzielt werden kann, so dass im Falle mangelnden Stimulationserfolges sehr kurzfristig ein Backup-Impuls auszulösen ist.

Bevorzugt ist eine Elektrostimulationsvorrichtung, bei der die Einrichtung zur Stimulationserfolgskontrolle ausgebildet ist, einen charakteristischen Abfall im zeitlichen Verlauf der erfassten Spannung oder einen Anstieg des zu Kurzschlussstromes oder eine entsprechende Veränderung der verknüpften Größe zu detektieren. Diese bevorzugte Einrichtung zur Stimulationserfolgskontrolle ist somit so gestaltet, dass sie einen Stimulationserfolg anhand eines kurzzeitigen und kurzfristigen, charakteristischen Abfalls der MyokardImpedanz detektiert, der zu einem entsprechenden Abfall der Kurzschlussspannung bzw. Anstieg des Kurzschlussstromes führt. Ein derartiger, kurzfristiger Abfall der Myokardimpedanz ist ein Anzeichen für eine erfolgreiche, d. h. überschwellige Stimulation des Herzmuskelgewebes. Mit den Worten "überschwellig" einerseits und "unterschwellig" andererseits werden Stimulationsimpulse charakterisiert, die einerseits eine ausreichende Intensität besitzen, um eine Kontraktion des Herzmuskelgewebes zu bewirken oder andererseits nicht von ausreichender Intensität zur erfolgreichen Reizung des Herzmuskelgewebes sind.

In einer bevorzugten Ausführungsvariante der Elektrostimulationsvorrichtung umfasst die Kapazität einen Koppelkondensator, der bei geschlossenem Kurzschlussschalter zwischen Elektrodenanschluss und Masseelektrode geschaltet ist. Ein solcher Koppelkondensator wird bei Herzschrittmachern üblicherweise verwendet, um nach Abgabe eines Stimulationsimpulses einen Stromfluss entgegengesetzter Richtung durch das Myokard zu bewirken, so dass eine reine Wechselstrom-Stimulation des Myocards erfolgt und Gleichströme vermieden werden. Der Koppelkondensator ist dazu während der Abgabe eines Stimulationsimpulses mit einem Reservoir-Kondensator und der Elektrode zur Abgabe des Stimulationsimpulses in Serie geschaltet. Der Reservoir-Kondensator enthält die Energie für den Stimulationsimpuls. Diese wird über den Koppelkondensator und Elektroden an das Myokard abgegeben. Dabei wird der Koppelkondensator über den Stimulationsimpuls geladen. Nach Abgabe des Stimulationsimpulses wird der Koppelkondensator über das Myokard kurzgeschlossen, so dass der zuvor erwähnte Stromfluss durch das Myokard eintritt, der dem Stromfluss während der Abgabe des Stimulationsimpulses entgegengerichtet ist. Dieser Stromfluss durch das Myokard nach Abgabe des Stimulationsimpulses wird von der Einrichtung zur Stimulationserfolgskontrolle erfasst und im Hinblick auf einen Impedanzeinbruch im Myokard ausgewertet.

Zu dem letztgenannten Zweck ist die Einrichtung zur Stimulationserfolgskontrolle vorzugsweise derart angeordnet und ausgebildet, dass sie die Spannung am Koppelkondensator erfasst.

Unabhängig von dem Vorhandensein eines Koppelkondensators, also auch dann, wenn die in Frage stehende Kapazität die Helmholzkapazität, die sich auf der Oberfläche der Stimulationselektrode ausbildet, ist die Einrichtung zur Stimulationserfolgskontrolle vorzugsweise derart angeschlossen, dass sie die Stromstärke des Kurzschlussstromes oder die an der Kapazität anliegende Spannung am Elektrodenanschluss erfasst.

Falls ein Koppelkondensator vorgesehen ist, ist die Einrichtung zur Stimulationserfolgskontrolle entsprechend derart angeschlossen, dass sie die Stromstärke des Kurzschlussstromes oder die an der Kapazität anliegende Spannung zwischen dem Koppelkondensator und dem Elektrodenanschluss erfasst.

Um die zuvor angesprochene, kurzfristige Verringerung der MyokardImpedanz als Anzeichen für eine erfolgreiche Stimulation zu detektieren, umfasst die Einrichtung zur Stimulationserfolgskontrolle vorzugsweise ein Differenzierglied zum Differenzieren der erfassten Spannung oder der erfassten Stromstärke. In einer besonders bevorzugten Ausführungsvariante ist dieses Differenzierglied mit einem Schwellwertdetektor derart verbunden, dass die Einrichtung zur Stimulationserfolgskontrolle einen über einen vorgegebenen Grenzwert liegenden Abfall der erfassten Spannung detektiert, und zwar vorzugsweise durch Detektieren einer Schwellwertunterschreitung der Ableitung der erfassten Spannung.

Besonders bevorzugt ist eine Ausführungsvariante der Einrichtung zur Stimulationserfolgskontrolle, die mit Hilfe des Differenzierliedes eine Ableitung der erfassten Spannung generiert und beispielsweise durch Division auf die erfasste Spannung normiert. Das auf diese Weise generierte Signal wird anschließend auf ein Schwellwertkriterium hin analysiert.

Unabhängig von den Details der Einrichtung zur Stimulationserfolgskontrolle ist diese Einrichtung vorzugsweise mit einem Timer verbunden, der mit Abgabe eines Stimulationsimpulses zu starten ist und die Zeitdauer bis zur Detektion eines Stimulationserfolges ermittelt. Diese Ausführungsvariante liegt der Erkenntnis zu Grunde, dass aus der Zeitdauer zwischen Abgabe eines Stimulationsimpulses und Eintritt des Stimulationserfolges abzuleiten ist, in welchem Maße ein Stimulationsimpuls überschwellig ist. Bei minimal ausreichender Stimulationsintensität ist der zeitliche Abstand zwischen Abgabe des Stimulationsimpulses und Eintritt des Stimulationserfolges größer als bei einer Stimulationsintensität, die erheblich größer ist als minimal erforderlich.

Entsprechend der zuvor dargestellten Erkenntnis wird einer Ausführungsform der Elektrostimulations-Vorrichtung bevorzugt, bei der der Timer ein der Zeitdauer zwischen Stimulationsimpuls-Abgabe und Eintreten des Stimulationserfolges entsprechendes Zeitsignal ausgibt und mit einer Stimulationsstärken-Steuereinheit verbunden ist, die auf das Zeitsignal anspricht und eine Einstellung der Stärke des Stimulationsimpulses (der Stimulationsimpuls-Intensität) in Abhängigkeit des Zeitsignals bewirkt.

In einer besonders bevorzugten Ausführungsvariante ist die Stimulationsstärken-Steuereinheit ausgebildet, die Intensität eines Stimulationsimpulses in Abhängigkeit von der bei Abgabe eines vorangegangenen Stimulationsimpulses durch den Timer gemessenen Zeit dergestalt zu steuern, dass diese Zeit innerhalb eines vorgegebenen Zeitfensters maximiert wird.

Im Zusammenhang mit allen zuvor beschriebenen Ausführungsvarianten ist es vorzugsweise vorgesehen, dass die Masse-Elektrode von einem Gehäuse der Elektrostimulations-Vorrichtung oder einer Teiloberfläche dieses Gehäuses gebildet wird.

Die Energiequelle der elektrischen Stimulationsvorrichtung umfasst vorzugsweise einen Reservoir-Kondensator und besonders bevorzugt eine Ladungspumpe zum Laden des Reservoir-Kondensators. Zur Steuerung des Ladens des Reservoir-Kondensators und der Abgabe des Stimulationsimpulses sind vorzugsweise Schalter vorgesehen, die derart ausgebildet und mit dem Reservoir-Kondensator zu verbinden sind, dass der Reservoir-Kondensator zum Laden mit der Ladungspumpe und zum Abgeben eines Stimulationsimpulses mit den Elektrodenanschlüssen verbunden ist. Weitere Details bevorzugter Ausführungsvarianten sind der nachfolgenden Beschreibung eines Ausführungsbeispiels zu entnehmen. Die Ausführungsbeispiele werden nachfolgend mit Bezug auf die Zeichnungen näher erläutert. Von den Zeichnungen zeigen:

Figur 1   eine schematische Darstellung der wesentlichen Bestandteile eines Elektrostimulationsgerätes sowie ein elektrisches Ersatzschaltbild für eine an das Stimulationsgerät angeschlossene Elektrodenleitung und das mit dem Elektrostimulationsgerät und der Elektrodenleitung in Verbindung stehende Myokard;

Figur 2   eine schematische Darstellung einer Einrichtung zur Stimulationserfolgskontrolle des Elektrostimulationsgerätes aus Figur 1;

Figur 3a   ein Beispiel für ein bei erfolgreicher Stimulation am Eingang der Einrichtung zur Stimulationserfolgskontrolle anliegendes Signal;

Figur 3b   eine vergrößerte Darstellung eines Ausschnitts des Signals aus Figur 3a und

Figur 4   Beispiele für innerhalb der Einreichung zur Stimulationserfolgskontrolle aus dem Signal gemäß Figur 3 abgeleitete Signale.

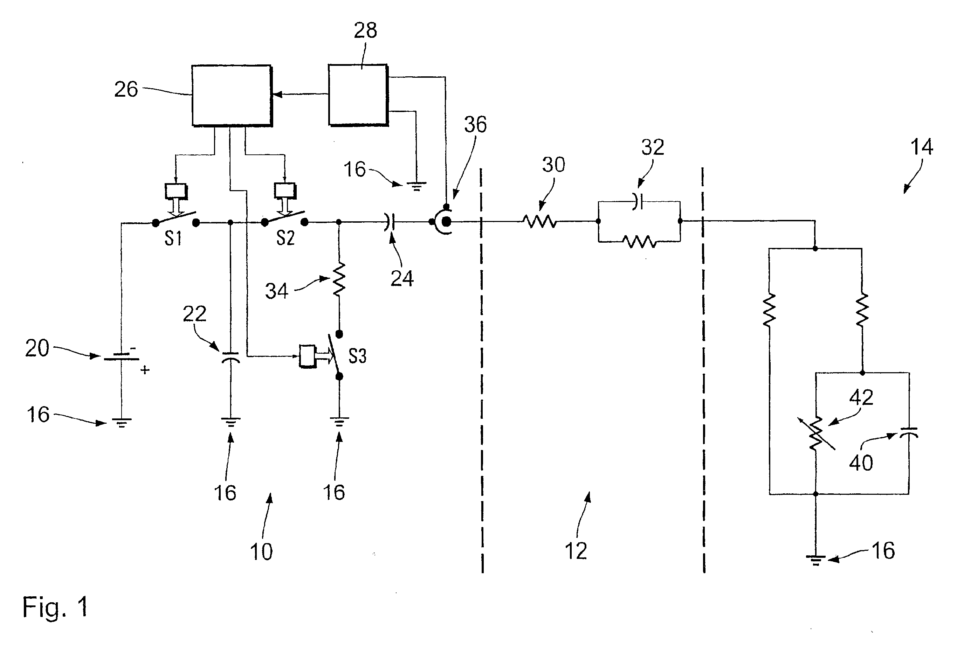

In Figur 1 links von der linken gestrichelten Linie dargestellt ist ein schematischer Blick über ein Elektrostimulationsgerät wie beispielsweise ein implantierbarer Herzschrittmacher, Kardioverter oder Defibrillator.

Zwischen den beiden gestrichelten Linien dargestellt ist ein elektrisches Ersatzschaltbild für eine an das Elektrostimulationsgerät 10 angeschlossene Elektrodenleitung 12.

Rechts von der rechten gestrichelten Linie in Figur 1 ist ein Ersatzschaltbild für das Herzmuskelgewebe 14 (Myokard) dargestellt, welches elektrisch mit einer Elektrode der Elektrodenleitung 12 und einer neutral - oder Masseelektrode 16 des Elektrostimulationsgerätes 10 verbunden ist.

Die wesentlichen Bestandteile des Elektrostimulationsgerätes 10 sind eine Energiequelle 20, die üblicherweise eine in der Figur nicht dargestellte Ladungspumpe enthält, ein Reservoir-Kondensator 22, ein Koppelkondensator 24, eine Steuereinheit 26 und eine Einrichtung 28 zur Stimulationserfolgskontrolle.

Die Energiequelle 20 ist in der Regel als Ladungspumpe ausgeführt, mit der eine einstellbare Spannung bereitgestellt werden kann, die auch über der Ausgangsspannung einer Versorgungsbatterie liegen kann. Das Symbol in der Zeichnung repräsentiert somit eine Versorgungsbatterie mit nachgeschalteter Ladungspumpe.

Der Reservoir-Kondensator 22 ist über einen Schalter S1 mit der Energiequelle 20 so zu verbinden, dass er parallel zur Energiequelle 20 geschaltete ist und durch diese geladen wird.

Außerdem ist der Reservoir-Kondensator 22 über einen Schalter S2 über den Koppel-Kondensator 24 mit der Elektrodenleitung 12 zur Abgabe eines Stimulationsimpulses an das Myokard 14 zu verbinden.

Die Elektrodenleitung weist neben einem ohmschen Elektrodenleitungswiderstand 30 eine Helmholzkapazität 32 auf, die sich in bekannter Weise auf der Oberfläche einer Stimulationselektrode ausbildet. Bei der Abgabe eines Elektrostimulationsimpulses bei geschlossenem Schalter S2 und geöffneten Schaltern S1 und S3 sind der Koppelkondensator 24 in die Helmholtz-Kapazität 32 in Serie geschaltet.

Vor Abgabe eines Elektrostimulationsimpulses an das Myokard 14 wird zunächst der Reservoir-Kondensator 22 durch Schließen des Schalters S1 geladen. Anschließend wird der Schalter S1 geöffnet und der Schalter S2 zur Abgabe des Elektrostimulationsimpulses geschlossen. Der Reservoir-Kondensator 22 entlädt sich dann über den Koppelkondensator 24 und die Elektrodenleitung 12 mit ihrer Helmholzkapazität 32 sowie über das Myokard 14. Der Reservoir-Kondensator 22 ist dazu einerseits mit der Elektrodenleitung 12 verbunden, andererseits mit einer Neutral-Elektrode, die als Masse-elektrode 16 ausgebildet ist und mit dem Myokard 14 in elektrischer Verbindung steht. Die Neutral-Elektrode kann beispielsweise von einem Herzschrittmachergehäuse gebildet sein, sodass der Elektrostimulationsimpuls unipolar abgegeben wird. Die Neutral-Elektrode kann aber auch von einer separaten Elektrode an der Elektrodenleitung 12 gebildet sein, sodass der Elektrostimulationsimpuls bipolar abgegeben wird.

Nach Abgabe des Elektrostimulationsimpulses wird der Schalter S2 wieder geöffnet und der Schalter S3 geschlossen. Auf diese Weise ist der Koppelkondensator 24 über einen ohmschen Widerstand 34 und die Neutral-Elektrode 16 einerseits sowie über die Elektrodenleitung 12 und das Myokard 14 andererseits kurzgeschlossen. Dieser sogenannte Autoshort der Stimulationselektrode durch Schließen des Schalters S3 bewirkt ein Entladen der vom Interface zwischen Elektrode 16 und Körpergewebe gebildeten Helmholtz-Kapazität 32 und ermöglicht ein Sensing nach der Abgabe des Stimulationsimpulses. Als Sensing wird dabei das Erfassen elektrischer Potentiale im Myocard bezeichnet, wie sie beispielsweise auch für eine intrakardiales Elektrokardiogramm aufgenommen werden.

Zur Abgabe eines Elektrostimulationsimpulses werden somit die Schalter S1, S2 und S3 in dieser Reihenfolge nacheinander geschlossen und wieder geöffnet, bevor der nächste Schalter geschlossen wird. Das Schließen und Öffnen der Schalter S1, S2 und S3 wird durch die Steuereinheit 26 bewirkt.

Die Einrichtung 28 zur Stimulationserfolgskontrolle ist elektrisch mit einem Elektrodenleitungsanschluss 36 verbunden, der sich zwischen dem Koppelkondensator 24 und der Elektrodenleitung 12 befindet. Die Einrichtung 28 zur Stimulationserfolgskontrolle ist ausgebildet, die an dem Elektrodenleitungsanschluss 36 anliegende Spannung gegenüber dem Massepotential zu erfassen und ist hierzu mit der Neutral-Elektrode 16 verbunden.

Das Erfassen der Spannung am Elektrodenleitungsanschluss 36 zum Zwecke der Stimulationserfolgskontrolle erfolgt nach Abgabe des Elektrostimulationsimpulses bei geöffnetem Schalter S2 und geschlossenem Schalter S3.

Konkret wird beispielsweise zum Zeitpunkt t=1s ein Stimulus von 2.4V abgegeben, d. h. S2 wird geschlossen. Der Stimulus ist 0,5 ms lang, dann wird S2 wieder geöffnet und gleichzeitig S3 zum Autoshort geschlossen. Die Autoshortzeit ist 10 ms lang, danach ist S3 wieder offen. Während der Entladung erfolgt nach 5 ms eine Depolarisation, der Membranwiderstand ändert sich für 1 ms schlagartig. Im Spannungsverlauf äußert sich dies als eine veränderte Zeitkonstante der Entladung. Nach der Depolarisation entlädt sich die Elektrode mit der ursprünglichen Zeitkonstanten aber auf einem anderen Niveau. Die Kurve macht also einen kleinen Sprung.

Die Einrichtung 28 zur Stimulationserfolgskontrolle ist mit der Steuereinheit 26 verbunden und gibt an die Steuereinheit 26 ein jeweils anderes Signal aus, je nachdem ob die Einrichtung 28 zur Stimulationserfolgskontrolle eine erfolgreiche Elektrostimulation detektiert hat oder nicht. Liegt nach Abgabe eines Stimulationsimpulses seitens der Einrichtung 28 zur Stimulationserfolgskontrolle kein Signal am entsprechenden Eingang der Steuereinheit 26 an, bewirkt die Steuereinheit 26 eine automatische Anpassung der Stimulationsimpulsintensität durch Erhöhen der Impulsspannung oder durch Verlängern der Impulsdauer.

Die Steuereinrichtung 26 kann dabei so ausgebildet sein, dass bei Ausbleiben eines einen Stimulationserfolg kennzeichnenden Signals seitens der Einrichtung 28 zur Stimulationserfolgskontrolle sofort ein zweiter Stimulationsimpuls größere Energie als Back-Up-Impuls abgegeben wird.

Die Detektion eines Stimulationserfolges durch die Einrichtung 28 zur Stimulationserfolgskontrolle geschieht durch Auswerten der am Elektrodenleitungsanschluss 36 anliegenden Spannung. Diese bricht bei einer erfolgreichen Elektrostimulation kurzfristig ein, da sich die Impedanz des Myokards - in Figur 1 dargestellt durch eine Parallelschaltung einer Kapazität 40 und eines veränderlichen ohmschen Widerstandes 42 - bei erfolgreicher Stimulation kurzzeitig dahingehend ändert, dass der ohmsche Widerstand 42 des Myokards für einen Moment abnimmt, weil sich die Ionenkanäle, insbesondere die Natrium-Kanäle der Muskelzellen, öffnen. Dieser Impedanz-Dip wird durch die Einrichtung 28 zur Stimulationserfolgskontrolle erfasst und dahingehend ausgewertet, dass die Einrichtung 28 zur Stimulationserfolgskontrolle ein einen Stimulationserfolg charakterisierendes Signal an die Steuereinheit 26 ausgibt.

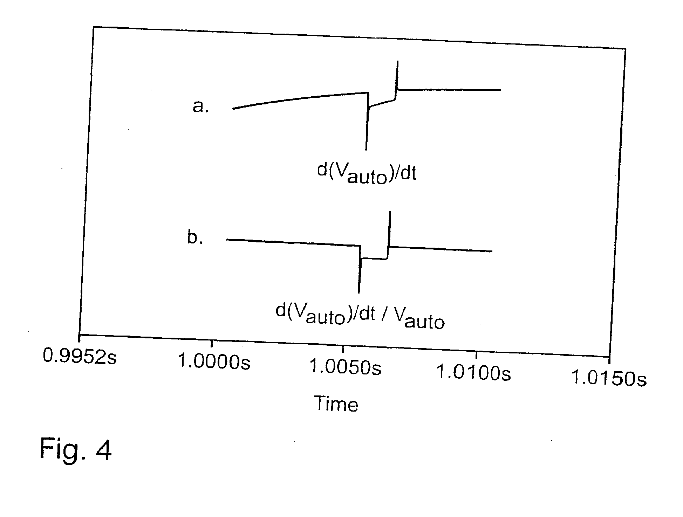

Da die Effekte der Membranwiderstandsänderung klein sind, kann die Depolarisation möglicherweise nur schwer aus dem Verlauf der Entladespannung selbst erkannt werden. Um den Sprung der Zeitkonstanten besser zu erkennen, kann die Kurve abgeleitet werden (Figur 4, Kurve a) oder die differenzierte Kurve zusätzlich noch durch die Ausgangskurve dividiert werden (Figur 4, Kurve b).

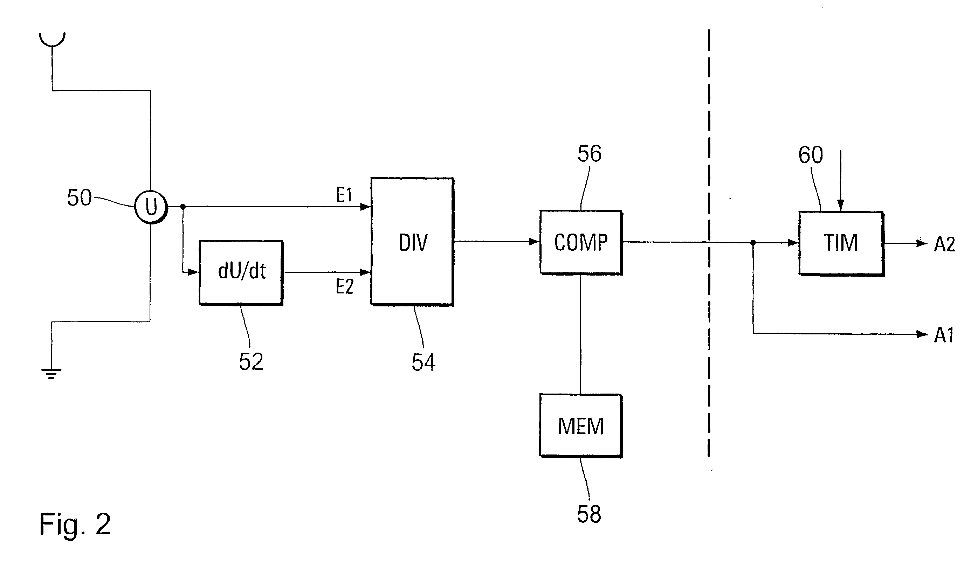

In einer bevorzugten Ausführungsvariante erfasst die Einrichtung 28 zur Stimulationserfolgskontrolle sowohl die Spannung zwischen Neutral-Elektrode 16 und Elektrodenleitungsanschluss 36 über eine Spannungsmesseinheit 50 (siehe Figur 2) als auch die Ableitung dieser Spannung nach der Zeit. Dazu ist der Spannungsmesseinheit 50 ein Differenzierglied 52 nachgeschaltet. Über ein Dividierglied 54 wird die Ableitung der erfassten Spannung auf die erfasste Spannung normiert. Dazu wird dem Dividierglied 54 sowohl das Ausgangssignal des Differenzierglieds 52 an einem Eingang E2 als auch die von der Spannungsmesseinheit 50 erfasste Spannung an einem Eingang E1 des Dividergliedes 54 bereitgestellt. Im Dividierglied 54 wird der Wert am Eingang E 2 (die Ableitung der Spannung nach der Zeit) durch den Wert am Eingang E1 (die erfasste Spannung) geteilt. Das am Ausgang des Dividergliedes 54 anliegende Signal wird einem Schwellwertdetektor zugeführt, der von einem Komperator 56 und einem Schwellwertspeicher 58 gebildet ist.

Überschreitet der Ausgangswert des Dividergliedes 54 den im Speicher 58 gespeicherten Vergleichswert, ist dies ein Anzeichen für eine erfolgreiche Elektrostimulation. Entsprechend liegt am Ausgang des Komperators 56, der gleichzeitig der Ausgang der Einrichtung 28 zur Stimulationserfolgskontrolle ist, ein einen Stimulationserfolg charakterisierendes Signal an.

Durch Analysieren der Ableitung der an der Einrichtung zur Stimulationserfolgskontrolle anliegenden Spannung wird de facto auch die Zeitkonstante für die Entladung der Koppelkapazität 24 und/oder der Helmholzkapazität 32 bestimmt, die sich als $\tau = R \cdot C$, also aus dem Produkt der jeweiligen Kapazität mit ohmschen Gewebewiderstand plus eventueller weiterer Widerstände wie dem Widerstand 34 bemisst.

Die Einrichtung 28 zur Stimulationserfolgskontrolle ist somit in der bevorzugten Ausführungsvariante dazu ausgebildet, die Zeitkonstante der bei geschlossenem Kurzschlussschalter S3 wirksamen R-C-Schaltung zu bestimmen und durch Schwellwertvergleich auszuwerten.

In einzelnen Fall, wenn man annimmt, dass alle die Entladung bestimmenden Widerstände und Kapazitäten in einer Zeitkonstanten T zusammengefasst sind, ergibt sich für die Autoshort-Spannung

$$V_{auto}(t) = V_0\, e^{-\frac{t}{T}} \cdot \quad \text{D. h.} \quad \frac{dV_{auto}}{dt}\frac{1}{V_{auto}} = -\frac{1}{T}$$

ist somit umgekehrt proportional zur Zeitkonstanten. Nach dieser Umrechnung des Signals kann eine Veränderung der Zeitkonstanten, z. B. mit einem adaptiven Schwellwertverfahren bestimmt werden. Mit solch einem Verfahren wird z. B. ein gleitender Mittelwert der abgeleiteten und normierten Kurve gebildet. Überschreitet ein Wert dieser Kurve den gleitenden Mittelwert um einen bestimmten Schwellwert, wird eine Änderung der Zeitkonstanten erkannt.

Ein Stimulus wird somit als effektiv erkannt, wenn eine Veränderung der Entladungskonstanten in einem bestimmten Zeitfenster detektiert wird. Der Stimulus wird als nicht effektiv bewertet, wenn diese Änderung ausbleibt. In diesem Fall wird ein Sicherheitspuls abgegeben und entsprechend einem Capture Control-Algorithmus z. B. die Pulsamplitude erhöht.

In einer besonders bevorzugten Ausführungsvariante ist seitens der Steuereinheit 26 ein Timer 60 vorgesehen, der mit Abgabe eines Elektrostimulationsimpulses gestartet wird und mit Eintreffen eines einen Stimulationserfolg charakterisierenden Signals seitens der Einrichtung 28 zur Stimulationserfolgskontrolle wieder gestoppt wird. Die auf diese Weise von dem Timer 60 erfasst Zeit ist ein Maß dafür, wie stark ein Elektrostimulationsimpuls überschwellig ist also um wie viel der Elektrostimulationsimpuls über der Reizschwelle des Myokards liegt. Bei nur schwach überschwelligen Stimulationsimpuls ist die vom Timer 60 erfasste Zeit zwischen Abgabe eines Elektrostimulationsimpulses und Eintreten des Stimulationserfolges länger, als bei einem stark überschwelligen Stimulationsimpuls.

Die Steuereinheit 26 ist daher vorzugsweise so ausgebildet, dass sie zur Energieeinsparung eine Absenkung der Stimulationsimpulsintensität bewirkt, wenn die von dem Timer 60 erfasste Zeit besonders kurz ist, beispielsweise kürzer als eine Millisekunde.

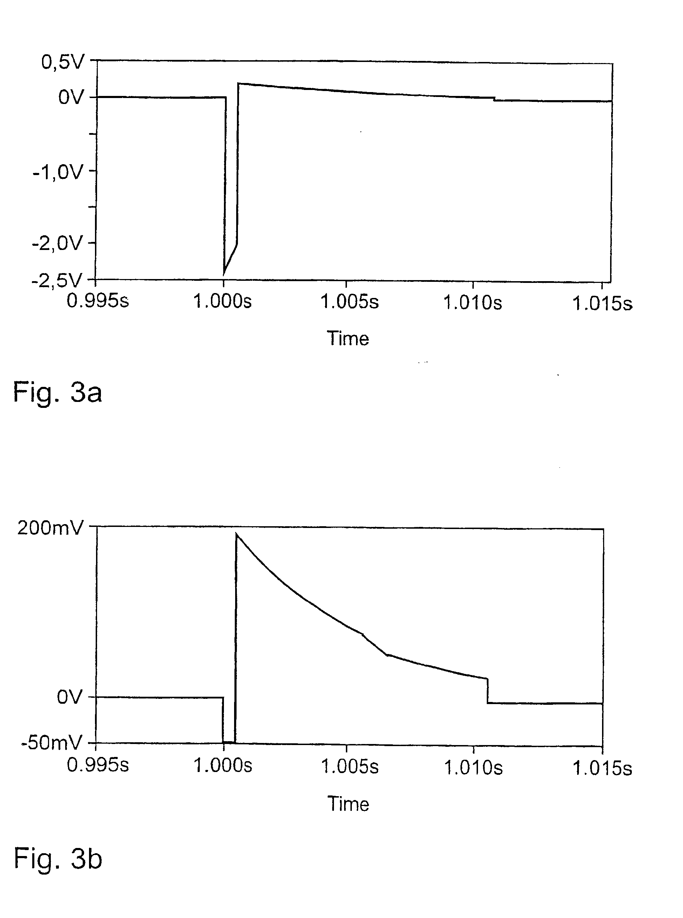

Die Figuren 3 a und b zeigen beispielhaft die von der Einrichtung 28 zur Stimulationserfolgskontrolle erfasste Spannung bei erfolgreicher Elektrostimulation. Figur 3b ist dabei eine vergrößerte Darstellung eines Ausschnittes aus Figur 3 a.

Figur 4 zeigt das Signal, welches am Ausgang des Differenziergliedes 52 (Kurve a) bzw. am Ausgang des Dividergliedes 54 (Kurve b) anliegt.

**Patentansprüche**

1. Vorrichtung zur Elektrostimulation von Körpergewebe, insbesondere Herzschrittmacher,

      - mit einem Energiespeicher (22) zur Bereitstellung elektrischer Stimulationsenergie,
      - mit einem Elektroden-Anschluss (36) zum Anschließen einer Stimulationselektrode (12) für die Abgabe von elektrischen Stimulationsimpulsen an Körpergewebe (16)
- mit einem ersten Schalter (S2), mit welchem der Energiespeicher (22) zur Abgabe eines Stimulationsimpulses mit dem Elektroden-Anschluss (36) zu verbinden ist,
- mit einer Einrichtung (28) zur Stimulationserfolgskontrolle,
- mit einem Kurzschluss-Schalter (S3), mit dem der Elektroden-Anschluss (36) nach Abgabe des Simulationsimpulses mit einem Massepotential (16) so zu verbinden ist, dass sich im Falle einer angeschlossenen und implantierten Elektrodenleitung (12) eine Kapazität (24, 32) über das Körpergewebe (14) und eine Masse-Elektrode (16) derart entladen kann, dass ein Kurzschlussstrom durch das Körpergewebe (14) fließt, wobei die Kapazität eine Helmholtz-Kapazität (32) einschließt, welche sich auf der Oberfläche der Stimulationselektrode (12) in Verbindung mit umgebender Körperflüssigkeit oder Körpergewebe einstellt,
- sowie mit einer Steuereinheit (26), die mit dem Schalter (S2) und dem Kurzschlussschalter (S3) zum Umschalten der Schalter (S2, S3) verbunden und ausgebildet ist, den Elektrodenleitungsanschluss (36) nach Abgabe des Stimulationsimpulses durch Öffnen des Schalters (S2) von der Energiequelle (20) zu trennen und durch Schließen des Kurzschluss-Schalters (S3) die Kapazität (24, 32) mit dem Massepotential (16) so zu verbinden dass sie über das Körpergewebe (14) kurzgeschlossen ist,

**dadurch gekennzeichnet, dass** die Einrichtung (28) zur Stimulationserfolgskontrolle nach Abgabe eines Stimulationsimpulses mit dem Elektrodenanschluss (36) verbunden ist und ausgebildet ist, nach Abgabe des Stimulationsimpulses bei geöffnetem Schalter (S2) und geschlossenem Kurzschluss-Schalter (S3), den zeitlichen Verlauf einer an der Kapazität (24; 32) über das Körpergewebe 14 anliegenden Kurzschlussspannung oder Kurzschlussstrom oder einer mit einer dieser Größen verknüpften Größe zu erfassen.

2. Elektrostimulations-Vorrichtung nach Anspruch 1, **dadurch gekennzeichnet, dass** die Einrichtung (28) zur Stimulationserfolgskontrolle ausgebildet ist, einen charakteristischen Abfall im zeitlichen Verlauf der erfassten Kurzschlussspannung oder einen Anstieg des Kurzschlussstromes eine entsprechende Veränderung der verknüpften Größe zu detektieren.

3. Elektrostimulations-Vorrichtung nach Anspruch 1 oder 2, **dadurch gekennzeichnet, dass** die Kapazität einen Koppelkondensator (24) umfasst, der bei geschlossenem Kurzschlussschalter (S3) zwischen Elektroden-Anschluss (36) und Masse-Elektrode (16) geschaltet ist.

4. Elektrostimulations-Vorrichtung nach Anspruch 3, **dadurch gekennzeichnet, dass** der Koppelkondensator (24) zwischen dem Energiespeicher (22) und wenigstens einem Elektroden-Anschluss (36) derart angeordnet ist, dass der Koppelkondensator (24) bei geschlossenem ersten Schalter (S2) mit dem Energiespeicher (22) in Reihe geschaltet ist.

5. Elektrostimulations-Vorrichtung nach Anspruch 3 oder 4, **dadurch gekennzeichnet, dass** die Einrichtung (28) zur Stimulationserfolgskontrolle angeordnet und ausgebildet ist, die Kurzschlussspannung am Koppelkondensator (24) zu erfassen.

6. Elektrostimulations-Vorrichtung nach einem der Ansprüche 1 bis 5, **dadurch gekennzeichnet, dass** die Einrichtung (28) zur Stimulationserfolgskontrolle derart angeschlossen ist, dass sie die Stromstärke des Kurzschlussstromes oder die an der Kapazität anliegende Kurzschlussspannung am Elektroden-Anschluss (36) erfasst.

7. Elektrostimulations-Vorrichtung nach Anspruch 3 und einem der Ansprüche 1, 2, 4, 5 oder 6, **dadurch gekennzeichnet, dass** die Einrichtung (28) zur Stimulationserfolgskontrolle derart angeschlossen ist, dass sie die Stromstärke des Kurzschlussstromes oder die an der Kapazität anliegende Kurzschlussspannung zwischen dem Koppelkondensator (24) und dem Elektroden-Anschluss (36) erfasst.

8. Elektrostimulations-Vorrichtung nach einem der Ansprüche 1 bis 7, **dadurch gekennzeichnet, dass** die Einrichtung (28) zur Stimulationserfolgskontrolle ein Differenzierglied (52) zum Differenzieren der erfassten Kurzschlussspannung oder der erfassten Kurzschlussstromstärke umfasst.

9. Elektrostimulations-Vorrichtung nach Anspruch 8, **dadurch gekennzeichnet, dass** das Differenzierglied (52) mit einem Schwellwertdetektor derart verbunden ist, dass die Einrichtung (28) zur Stimulationserfolgskontrolle einen über einem vorgegebenen Grenzwert liegenden Abfall der erfassten Kurzschlussspannung detektiert.

10. Elektrostimulations-Vorrichtung nach Anspruch 9, **dadurch gekennzeichnet, dass** das Differenzierglied (52) mit einem Schwellwertdetektor derart verbunden ist, dass die Einrichtung (28) zur Stimulationserfolgskontrolle eine Schwellwertunterschreitung der Ableitung der erfassten Kurzschlussspannung ermittelt.

11. Elektrostimulations-Vorrichtung nach Anspruch 9 oder 10, **dadurch gekennzeichnet, dass** das Differenzierglied (52) mit einem Schwellwertdetektor derart verbunden ist, dass die Einrichtung (28) zur Stimulationserfolgskontrolle eine Schwellwertunterschreitung der Ableitung der erfassten Kurzschlussspannung normiert auf die erfasste Kurzschlussspannung detektiert.

12. Elektrostimulations-Vorrichtung nach einem der Ansprüche 1 bis 11, **dadurch gekennzeichnet, dass** die Einrichtung (28) zur Stimulationserfolgskontrolle mit einem Timer (60) verbunden ist, der mit Abgabe eines Stimulationsimpulses zu starten ist und die Zeit bis zur Detektion eines Stimulationserfolges ermittelt.

13. Elektrostimulations-Vorrichtung nach Anspruch 12, **dadurch gekennzeichnet, dass** der Timer (60) ein der Zeitdauer zwischen Stimulationsimpuls-Abgabe und Eintreten des Stimulationserfolges entsprechendes Zeitsignal ausgibt und mit einer Stimulationsstärken-Steuereinheit (26) verbunden ist, die auf das Zeitsignal anspricht und eine Einstellung der Stärke des Stimulationsimpulses in Abhängigkeit des Zeitsignals bewirkt.

14. Elektrostimulations-Vorrichtung nach einem der Ansprüche 1 bis 13, **dadurch gekennzeichnet, dass** die Masse-Elektrode (16) von einem Gehäuse der Elektrostimulations-Vorrichtung oder einer Teiloberfläche des Gehäuses gebildet ist.

15. Elektrostimulations-Vorrichtung nach einem der Ansprüche 1 bis 14, **dadurch gekennzeichnet, dass** der Energiespeicher wenigstens einen Reservoir-Kondensator (22) umfasst.

16. Elektrostimulations-Vorrichtung nach Anspruch 15, **dadurch gekennzeichnet, dass** eine Energiequelle (20) mit einer Ladungspumpe zum Laden des Reservoir-Kondensators (22) vorgesehen ist.

17. Elektrostimulations-Vorrichtung nach Anspruch 15 und 16, **dadurch gekennzeichnet, dass** die Energiequelle (20) Schalter (S2; S3) umfasst, die derart ausgebildet und mit dem Kondensator verbinden sind, dass der Reservoir-Konsensator (22) zum Laden mit der Ladungspumpe und zum Abgeben eines Stimulationsimpulses mit den Elektrodenanschlüssen verbunden ist.

## Claims

1. A device for the electrostimulation of body tissue, in particular a pacemaker, comprising:

   - an energy storage element (22) for providing electrical stimulation energy;
   - an electrode terminal (36) for connecting a stimulation electrode (12) for delivering electrical stimulation pulses to body tissue (16);
   - a first switch (S2) by way of which the energy storage element (22) is to be connected to the electrode terminal (36) for the delivery of a stimulation pulse;
   - a unit (28) for capture verification;
   - a short circuiting switch (S3) by way of which, after the delivery of the stimulation pulse, the electrode terminal (36) is to be connected to a ground potential (16) in such a way that, when an electrode lead (12) is connected and implanted, a capacitance (24, 32) can be discharged via the body tissue (14) and a ground electrode (16) in such a way that a short circuit current flows through the body tissue (14), wherein the capacitance includes a Helmholtz capacitance (32), which develops on the surface of the stimulation electrode (12) in conjunction with surrounding body fluid or body tissue;
   - and a control unit (26), which is connected to the switch (S2) and the short circuiting switch (S3) for switching the switches (S2, S3) and designed to disconnect the electrode lead terminal (36) from the energy source (20) after delivery of the stimulation pulse, by opening of the switch (S2), and to connect the capacitance (24, 32) to the ground potential (16), by closing of the short circuiting switch (S3), in such a way that the capacitance is short circuited via the body tissue (14),

   **characterized in that** the unit (28) for capture verification is connected to the electrode terminal (36), after delivery of a stimulation pulse, and is designed, after delivery of the stimulation pulse, with switch (S2) being open and short circuiting switch (S3) being closed, to detect the temporal progression of a short circuit voltage or short circuit current present at the capacitance (24; 32) via the body tissue, or to detect the temporal progression of a variable that is associated with one of these variables.

2. The electrostimulation device according to claim 1, **characterized in that** the unit (28) for capture verification is designed to detect a characteristic drop in the temporal progression of the detected short circuit voltage, or an increase in the short circuit current, or a corresponding change of the associated variable.

3. The electrostimulation device according to claim 1 or 2, **characterized in that** the capacitance includes a coupling capacitor (24), which is connected between the electrode terminal (36) and the ground electrode (16) when the short circuiting switch (S3) is closed.

4. The electrostimulation device according to claim 3, **characterized in that** the coupling capacitor (24) is disposed between the energy storage element (22) and at least one electrode terminal (36) in such a way that the coupling capacitor (24) is connected in series with the energy storage element (22) when the first switch (S2) is closed.

5. The electrostimulation device according to claim 3 or 4, **characterized in that** the unit (28) for capture verification is disposed and designed to detect the short circuiting voltage at the coupling capacitor (24).

6. The electrostimulation device according to any one of claims 1 to 5, **characterized in that** the unit (28) for capture verification is connected in such a way that it detects the current intensity of the short circuit current or the short circuit voltage present at the capacitance on the electrode terminal (36).

7. The electrostimulation device according to claim 3 and one of claims 1, 2, 4, 5 or 6, **characterized in that** the unit (28) for capture verification is connected in such a way that it detects the current intensity of the short circuit current or the short circuit voltage present at the capacitance between the coupling capacitor (24) and the electrode terminal (36).

8. The electrostimulation device according to any one of claims 1 to 7, **characterized in that** the unit (28) for capture verification includes a differentiating member (52) for differentiating the detected short circuit voltage or the detected short circuit current intensity.

9. The electrostimulation device according to claim 8, **characterized in that** the differentiating member (52) is connected to a threshold value detector in such a way that the unit (28) for capture verification detects a drop of the detected short circuit voltage which is above a predefined threshold value.

10. The electrostimulation device according to claim 9, **characterized in that** the differentiating member (52) is connected to a threshold value detector in such a way that the unit (28) for capture verification ascertains a drop of the derivative of the detected short circuit voltage below a threshold value.

11. The electrostimulation device according to claim 9 or 10, **characterized in that** the differentiating member (52) is connected to a threshold value detector in such a way that the unit (28) for capture verification detects a drop of the derivative of the detected short circuit voltage below a threshold value, normalized for the detected short circuit voltage.

12. The electrostimulation device according to any one of claims 1 to 11, **characterized in that** the unit (28) for capture verification is connected to a timer (60), which is to be started with the delivery of a stimulation pulse and ascertains the time until the detection of capture.

13. The electrostimulation device according to claim 12, **characterized in that** the timer (60) outputs a time signal corresponding to the time duration between the delivery of the stimulation pulse and the occurrence of capture and is connected to a stimulation intensity control unit (26), which responds to the time signal and causes an adjustment of the intensity of the stimulation pulse as a function of the time signal.

14. The electrostimulation device according to any one of claims 1 to 13, **characterized in that** the ground electrode (16) is formed by a housing of the electrostimulation device or a partial surface of the housing.

15. The electrostimulation device according to any one of claims 1 to 14, **characterized in that** the energy storage element comprises at least one reservoir capacitor (22).

**16.** The electrostimulation device according to claim 15, **characterized in that** an energy source (20) including a charge pump for charging the reservoir capacitor (22) is provided.

**17.** The electrostimulation device according to claim 15 and 16, **characterized in that** the energy source (20) comprises switches (S2; S3), which are designed and connected to the capacitor in such a way that the reservoir capacitor (22) is connected to the charge pump for charging and to the electrode terminals for delivering a stimulation pulse.


**Revendications**

**1.** Dispositif pour la stimulation électrique de tissus corporels, en particulier un stimulateur cardiaque,

- avec un accumulateur d'énergie (22) pour la mise à disposition d'une énergie de stimulation électrique,
- avec une borne d'électrode (36) pour le raccordement d'une électrode de stimulation (12) pour la délivrance d'impulsions de stimulation électriques à des tissus corporels (16),
- avec un premier interrupteur (S2) destiné à relier l'accumulateur d'énergie (22) à la borne d'électrode (36) pour la délivrance d'une impulsion de stimulation,
- avec un dispositif (28) pour le contrôle de l'efficacité de la stimulation,
- avec un interrupteur à court-circuit (S3) destiné à relier la borne d'électrode (36) à un potentiel de masse (16) suite à la délivrance de l'impulsion de stimulation, de sorte que dans le cas d'une ligne d'électrode (12) raccordée et implantée, une capacité (24, 32) peut se décharger par le tissu corporel (14) et une électrode de masse (16) de manière à ce qu'un courant de court-circuit traverse le tissu corporel (14), sachant que la capacité inclut une capacité de Helmholtz (32) qui s'établit à la surface de l'électrode de stimulation (12) en association avec un liquide corporel ou des tissus corporels environnants,
- ainsi qu'avec une unité de commande (26) reliée à l'interrupteur (S2) et l'interrupteur de court-circuit (S3) pour la commutation des interrupteurs (S2, S3), et conçue pour séparer la borne d'électrode (36) de la source d'énergie (20) en ouvrant l'interrupteur (S2) suite à la délivrance de l'impulsion de stimulation, et pour relier la capacité (24, 32) au potentiel de masse (16) en fermant l'interrupteur de court-circuit (S3) de manière à ce que celle-ci soit court-circuitée par le tissu corporel (14),

**caractérisé en ce que** le dispositif (28) pour le contrôle de l'efficacité de la stimulation est relié à la borne d'électrode (36) suite à la délivrance d'une impulsion de stimulation, et conçu pour détecter l'évolution dans le temps d'une tension de court-circuit ou d'un courant de court-circuit appliqué(e) à la capacité (24, 32) par le biais du tissu corporel (14) ou d'une valeur corrélée avec ces valeurs, suite à la délivrance de l'impulsion de stimulation, lorsque l'interrupteur (S2) est ouvert et l'interrupteur de court-circuit (S3) est fermé.

**2.** Dispositif pour la stimulation électrique selon la revendication 1, **caractérisé en ce que** le dispositif (28) pour le contrôle de l'efficacité de la stimulation est conçu pour détecter une baisse caractéristique dans l'évolution dans le temps de la tension de court-circuit détectée, ou une augmentation du courant de court-circuit ou une modification correspondante de la valeur corrélée.

**3.** Dispositif pour la stimulation électrique selon la revendication 1 ou 2, **caractérisé en ce que** la capacité comprend un condensateur de couplage (24) branché entre la borne d'électrode (36) et l'électrode de masse (16) lorsque l'interrupteur de court-circuit (S3) est fermé.

**4.** Dispositif pour la stimulation électrique selon la revendication 3, **caractérisé en ce que** le condensateur de couplage (24) est agencé entre l'accumulateur d'énergie (22) et au moins une borne d'électrode (36), de telle façon que le condensateur de couplage (24) est relié en série avec l'accumulateur d'énergie (22) lorsque le premier interrupteur (S2) est fermé.

**5.** Dispositif pour la stimulation électrique selon la revendication 3 ou 4, **caractérisé en ce que** le dispositif (28) pour le contrôle de l'efficacité de la stimulation est agencé et conçu pour détecter la tension de court-circuit sur le condensateur de couplage (24).

**6.** Dispositif pour la stimulation électrique selon l'une des revendications 1 à 5, **caractérisé en ce que** le dispositif (28) pour le contrôle de l'efficacité de la stimulation est raccordé de manière à détecter l'intensité de courant du courant de court-circuit ou la tension de court-circuit appliquée à la capacité sur la borne d'électrode (36).

**7.** Dispositif pour la stimulation électrique selon la revendication 3 et l'une des revendications 1, 2, 4, 5 ou 6, **caractérisé en ce que** le dispositif (28) pour le contrôle de l'efficacité de la stimulation est raccordé de manière à détecter l'intensité de courant du courant de court-circuit ou la tension de court-circuit appliquée à la capacité entre le condensateur de couplage (24) et la borne d'électrode (36).

**8.** Dispositif pour la stimulation électrique selon l'une des revendications 1 à 7, **caractérisé en ce que** le dispositif (28) pour le contrôle de l'efficacité de la stimulation comprend un organe de différentiation (52) pour différencier la tension de court-circuit détectée ou l'intensité détectée du courant de court-circuit.

**9.** Dispositif pour la stimulation électrique selon la revendication 8, **caractérisé en ce que** l'organe de différenciation (52) est relié de telle façon à un détecteur de valeur seuil, que le dispositif (28) pour le contrôle de l'efficacité de la stimulation détecte une baisse de la tension de court-circuit détectée au-delà d'une valeur seuil prédéfinie.

**10.** Dispositif pour la stimulation électrique selon la revendication 9, **caractérisé en ce que** l'organe de différentiation (52) est relié à un détecteur de valeur seuil de manière à ce que le dispositif (28) pour le contrôle de l'efficacité de la stimulation détermine si la dérivation de la tension de court-circuit détectée tombe en-dessous d'une valeur seuil.

**11.** Dispositif pour la stimulation électrique selon la revendication 9 ou 10, **caractérisé en ce que** l'organe de différenciation (52) est relié à un détecteur de valeur seuil de manière à ce que le dispositif (28) pour le contrôle de l'efficacité de la stimulation détecte si la dérivation de la tension de court-circuit détectée normalisée sur la tension de court-circuit détectée tombe en-dessous d'une valeur seuil.

**12.** Dispositif pour la stimulation électrique selon l'une des revendications 1 à 11, **caractérisé en ce que** le dispositif (28) pour le contrôle de l'efficacité de la stimulation est relié à un minuteur (60) destiné à être démarré lors de la délivrance d'une impulsion de stimulation et à déterminer la durée jusqu'à la détection d'une stimulation réussie.

**13.** Dispositif pour la stimulation électrique selon la revendication 12, **caractérisé en ce que** le minuteur (60) émet un signal temporel correspondant à la durée entre la délivrance d'une impulsion de stimulation et la réussite, tout en étant relié à une unité de commande d'intensités de stimulation (26) répondant au signal temporel et entraînant un réglage de l'intensité de l'impulsion de stimulation en fonction du signal temporel.

**14.** Dispositif pour la stimulation électrique selon l'une des revendications 1 à 13, **caractérisé en ce que** l'électrode de masse (16) est formée par un boîtier du dispositif pour la stimulation électrique ou par une surface partielle du boîtier.

**15.** Dispositif pour la stimulation électrique selon l'une des revendications 1 à 14, **caractérisé en ce que** l'accumulateur d'énergie comprend au moins un condensateur de réservoir (22).

**16.** Dispositif pour la stimulation électrique selon la revendication 15, **caractérisé en ce qu'**il est prévu une source d'énergie (20) avec une pompe de charge pour charger le condensateur de réservoir (22).

**17.** Dispositif pour la stimulation électrique selon la revendication 15 ou 16, **caractérisé en ce que** la source d'énergie (20) comprend des interrupteurs (S2 ; S3) conçus et reliés au condensateur de manière à ce que le condensateur de réservoir (22) soit relié à la pompe de charge pour la charge et aux bornes d'électrode pour la délivrance d'une impulsion de stimulation.

Fig. 1

EP 1 415 682 B1

Fig. 2

Fig. 3a

Fig. 3b

a.

$d(V_{auto})/dt$

b.

$d(V_{auto})/dt / V_{auto}$

0.9952s    1.0000s    1.0050s    1.0100s    1.0150s

Time

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5350410 A
- US 5411533 A
- US 5431639 A
- US 5674254 A
- US 5713933 A
- US 5735883 A
- US 5766230 A
- EP 0399063 A
- US 6226551 B